(19) Europäisches Patentamt / European Patent Office / Office européen des brevets

(11) **EP 3 296 017 A1**

(12) **EUROPEAN PATENT APPLICATION**

(43) Date of publication:
**21.03.2018 Bulletin 2018/12**

(51) Int Cl.:
**B01L 3/00** *(2006.01)*    **A61B 5/15** *(2006.01)*

(21) Application number: **16189473.8**

(22) Date of filing: **19.09.2016**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Vital Esthetique Sarl
75015 Paris (FR)**

(72) Inventor: **YOUSSEF, Ahmed
35858 Qadisiyah (KW)**

(74) Representative: **Ivanov, Ivan Nikolov
Mitropolit Kiril Vidinski Str.
No. 6-8, entr. 8, floor 2
1164 Sofia (BG)**

(54) **COLLECTION TUBE AND CALIBRATED TRANSPARENT TUBE FOR FRACTIONATION IN PROVISION OF PLATELETS RICH PLASMA**

(57)    The present invention relates to a tube for fractionating biological fluids in order to provide a platelets rich product, in particular a platelets rich plasma.

Platelet rich plasma (PRP) is the product of filtration of blood plasma, characterized by a high concentration of platelets. The utilization of such a filtered fraction has been performed in many therapeutic, cosmetic as well as laboratory evidence as medium for cell expansion in laboratory conditions.

A collection tube made of plastic material with a wide inner diameter and short length, preferably up to 20 cm, internally coated with a platelet anti-adhesive, characterized in that it is fitted with a needle / 2 / at one end of the tube / 1 / which is preferably 23- gauge sharp needle cannula with a butterfly-fixator for blood collection by venipuncture and needle / 3 / in the opposite end of the tube / 1 / which is 16-gauge sharp needle.

EP 3 296 017 A1

**Description**

**FIELD OF THE INVENTION**

The present invention relates to a tube for fractionating biological fluids in order to provide a platelets rich product, in particular a platelets rich plasma.

**BACKGROUND OF THE INVENTION**

Platelet rich plasma (PRP) is the product of filtration of blood plasma, characterized by a high concentration of platelets. The utilization of such a filtered fraction has been performed in many therapeutic, cosmetic as well as laboratory evidence as medium for cell expansion in laboratory conditions.

Furthermore, it is known that PRP plasma is a concentrated source of autonomous platelets that contains several growth factors, called cytokines, which stimulate the rapid healing of bone and soft tissue

It is known to all that human blood is composed of red blood cells, white blood cells, platelets and plasma. Platelets are involved in blood clotting process and are formed in the bone marrow.

Research from the last decades show that when activated in the body, platelets release proteins, called growth factors. They accelerate wound healing. Therefore, when increasing the concentration of blood platelets, treatment is able to provide a potent cocktail of growth factors, which could accelerate dramatically tissue repair, especially the tendons, as well as wound healing. Of greater than a decade using therapeutic and cosmetic effects of PRP as it serves to accelerate the healing of wounds and bones in orthopedic, cardiac and dental operations. In dermatology and plastic surgery PRP is widely used for healing of chronic ulcers (e.g. Diabetic leg ulcer) and an autologous rejuvenation with or without injections of fat

Patent application publication number US 20102/0251411 provides a tube, allowing the separation of specific components from the blood sample. According to the disclosure the tube has an outer and inner surface, the tube comprises an upper chamber provided with an open top and a constant internal volume, medium chamber, which is connected to the upper chamber at its lower end and having a smaller diameter relative to the upper chamber and a lower chamber associated with the intermediate chamber and having a larger diameter than the intermediate chamber and there is at least one supportive portion arranged integrally on the outer wall of the intermediate chamber.

It is important to note that there is a continuing need of provision a means for economical, safe and effective separation of biological substances, ensuring effective protection from contamination, including biological contamination arising during the process of separation in order to acquire sufficient fraction rich in platelets, in particular a PRP plasma

The three stages through which blood passes until it reaches the PRP are very crucial for achieving true value and impact of PRP. These three stages are:

(A) Collection of blood sample

(B) fractionation in a centrifugation tube

(C) Treatment for activation and re-injection

During the conduction of the abovementioned three stages there has always been factors of human error that may lead to poor PRP effects. The stages may be modified in order to insure the preservation of the therapeutic value of the acceleration PRP within the highest standards by discovering of an economically advantageous scheme

BRIEF DESCRIPTION OF THE DRAWINGS

Figure 1 - depicts a view of the flexible collection tube for collecting a blood sample together with the associated needles in both ends of the tube, according to the invention

Fig. 2 represents the fractional tube for centrifugal separation of platelet rich plasma, according to the invention

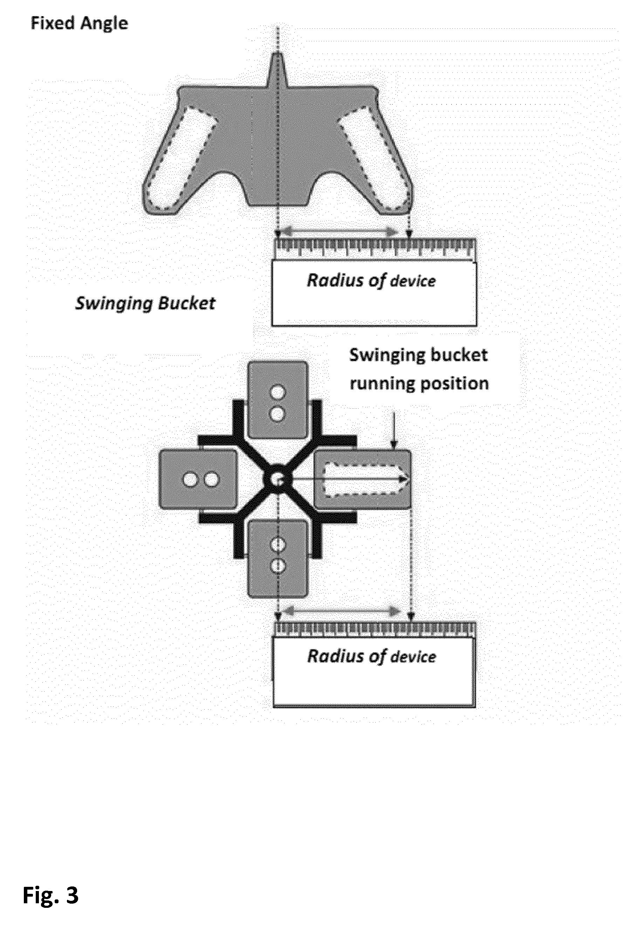

Fig. 3 - scheme for adjustment the angle of centrifugal devices with fixed and movable angle

Fig. 4 represents the calibrated transparent tube for separation of a plasma rich platelet immediately before centrifugation

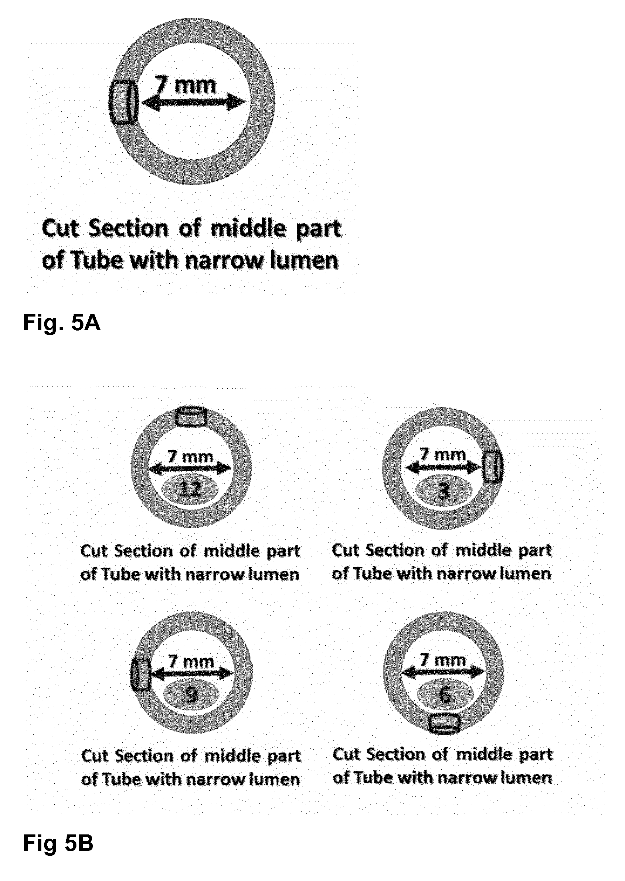

Fig. 5a - represents cross section of the middle chamber of the calibrated tube formed with an aperture provided with a stopper

Fig. 5b - represents cross section of the middle chamber of the calibrated tube, at various locations of the formed openings, provided with stoppers.

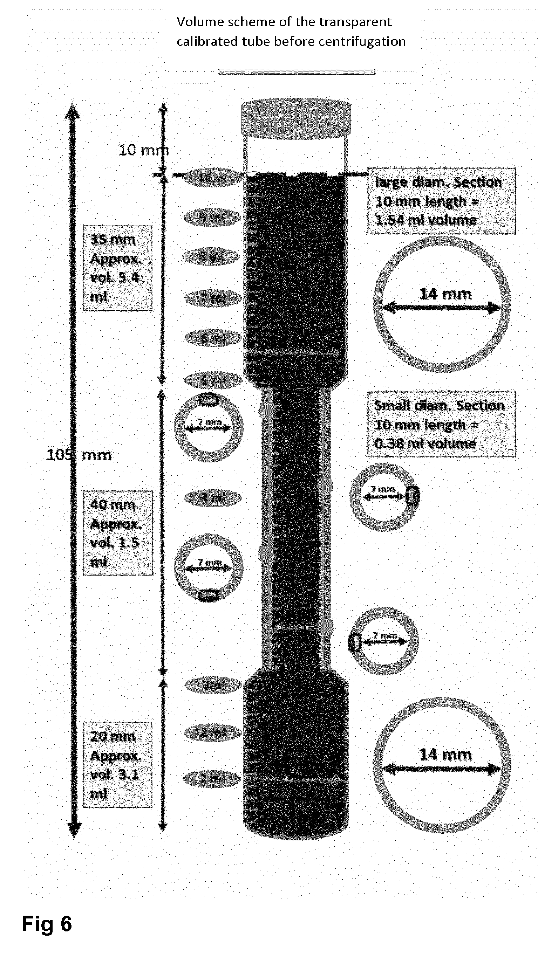

Fig. 6 - presents the volume scheme of the calibrated transparent tube/4/ before centrifugation

Figures 7a-c present different scenarios of view of the calibrated transparent tube/4/ after centrifugation for patients with different number and / or volume of red blood cells (RBCs)

DESCRIPTION OF THE INVENTION

For a clear and complete understanding of the invention, it will now be disclosed by preferred embodiments and by aligning it with the accompanying drawings and corresponding stages of the method as presented above.

For purposes of this invention the scheme of acquiring PRP passes through the following stages with the corresponding modification..

1 stage: statge of collection of blood sample

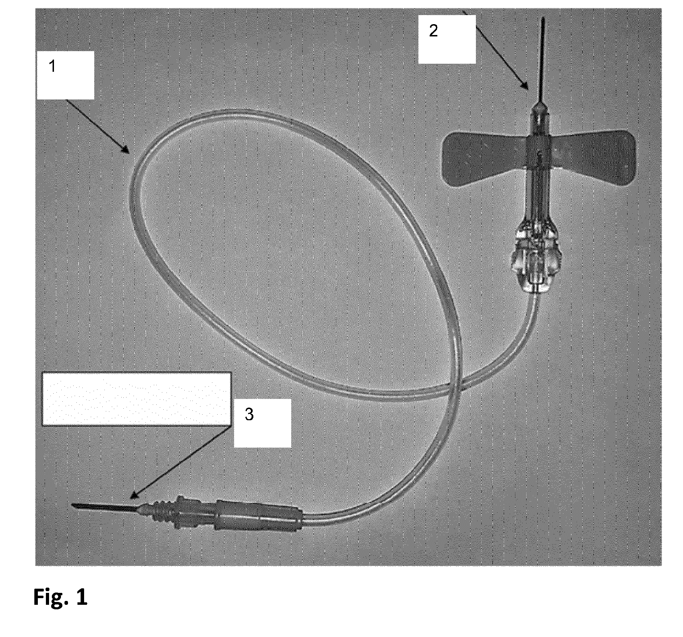

The collection of the blood sample is carried out by standard procedures known to those skilled in the art, but is modified by using a collection tube / 1 / in accordance with Figure 1, according to the invention, made of plastic material with a wide inner diameter and short length (preferably only 20 cm ), which is internally coated with a platelet anti-adhesive, to prevent adhesion of platelets and / or their degranulation during the process of blood collection

Furthermore, the tube / 1 / in accordance with Figure 1 is provided with two needles with a wider opening (compared to the needles for injection) at both ends:

The needle / 2 / at one end of the tube / 1 / according to Figure 1 is preferably 23 gauge sharp needle cannula with a butterfly-fixator for blood collection by venipuncture from the subject.

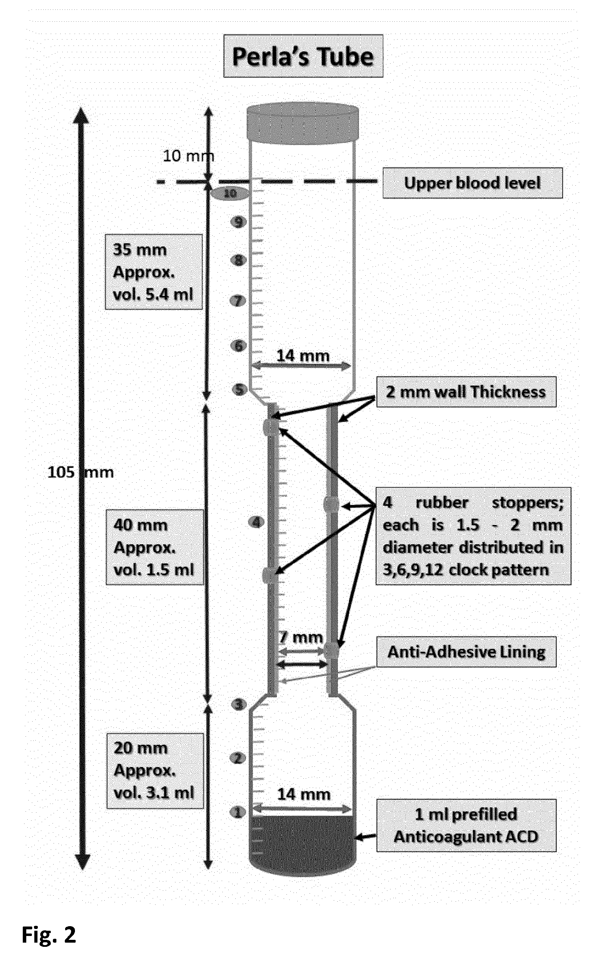

The needle / 3 / in the opposite end of the tube /1/ is 16 gauge sharp needle to drain the blood on top of a transparent calibrated tube / 4 / according to Figure 2, in which the blood will be centrifuged

2 stage is a stage of centrifugal separation ot PRP (platelets rich plasma) by calibrated transparent tube for fractionation

This stage of the process of acquiring of PRP fraction is conducted with a PRP (plasma rich in platelets) tube for fractionation, which is calibrated transparent tube / 4/ made up of three chambers- upper chamber / 4a /, middle chamber/ 4b / and a lower chamber / 4b, the middle chamber/ 4b / is equipped with openings / 5 /, all located on the wall / 6 / of the middle chamber / 4b / in the calibrated transparent tube / 4 / which allows to determine the level at which should be aspirated from the tube to remain accurate aspirated components. All openings / 6 / are provided with stoppers / 7 / made of flexible material, preferably rubber

It is important to note that according to the invention the calibrated transparent tube / 4 / is preferably of a length 105 mm and has a maximum diameter 15mm. These dimensions make it possible the tube / 4 / to fit in any centrifugal device known to those skilled in the art, eliminating the need to use a specific device for centrifugation, with a calibrated volume in its three chambers.

By providing a negative pressure in the transparent calibrated tube / 4 / allowing direct slow aspiration of 9ml blood without any blood exposure. The transparent calibrated tube / 4 / for conducting the stage 2 is pre-filled with anticoagulant, preferably in volume of about 1ml and preferably the anticoagulant would be acid citrate dextrose (ACD-A). The inclusion of an anti-coagulant results in reducing the pH from approximately 7.4 to approximately 6.5, which helps impairing activation of thrombin and contributes to the overall maintenance of platelet morphology. ACD-A anti-coagulant acts as an anticoagulant by the action of the citrate ion cheating free ionized calcium, thus making calcium unavailable for coagulation system

It is important to note that conventional needles where the aspiration is carried out from above, where there are no openings in the middle chamber does not guarantee an increased rate of platelets due to shake and the turbulent effect caused by aspiration from above as well as the difficulty of aspirate the platelets adhered to the wall. It has surprisingly been found that the aspiration openings of the side wall of the middle sector completely overcomes the occurrence of such problems

In the table below is shown an example of an anticoagulant composition containing acid citrate and dextrose

| ACD solution A (per 1000 ml) | |
| --- | --- |
| Total citrate (citric acid, anhydrous (C6H8O7) | 22 g |
| Dextrose (C6H12O6*H2O) | 25 g |
| Sodium (Na) | 5g |

Upon reaching the target amount of blood (i.e. 9ml), the cannula should be removed and discarded. The transparent calibrated tube / 4 / should be gently shaken in order to confirm mixing of the anticoagulant with blood and moved directly for centrifugation in centrifuge.

As mentioned above, the calibrated transparent tube / 4 / is designed so that it fits into any centrifugal device used and known to those skilled in the art.

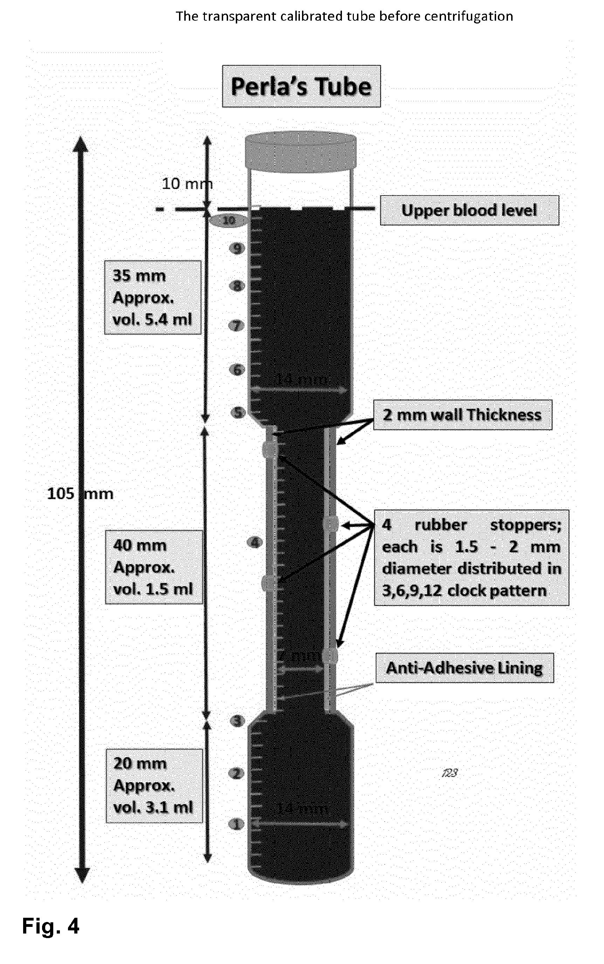

Prior to moving the transparent calibrated tube / 4 / for centrofugation, the radius of the centrifuge device should be measured, preferably as shown in Figure 4. It is important to know whether it is a centrifugal device with a fixed angle or a swinging bucket device, which are completely familiar to those skilled in the art.

Every specialist in the art knows that according to the radius, RPM settings could be determined if Centrifugation device is pprogrammed to ask for RPM or directly G-force will be used for settings, if it is included in the device settings

For a more complete understanding in the following table are represented roughly guidance for the conversion between the rpm and G- force

**Conversion Table**

| Speed (RPM) | Rotor Radius (from center of rotor to sample) in centimeters | | | | | | | | | | | |
|---|---|---|---|---|---|---|---|---|---|---|---|---|
| | 4 | 5 | 6 | 7 | 8 | 9 | 10 | 11 | 12 | 13 | 14 | 15 |
| 1000 | 45 | 56 | 67 | 78 | 89 | 101 | 112 | 123 | 134 | 145 | 157 | 168 |
| 1500 | 101 | 126 | 151 | 176 | 201 | 226 | 252 | 277 | 302 | 327 | 352 | 377 |
| 2000 | 179 | 224 | 268 | 313 | 358 | 402 | 447 | 492 | 537 | 581 | 626 | 671 |
| 2500 | 280 | 349 | 419 | 489 | 559 | 629 | 699 | 769 | 839 | 908 | 978 | 1048 |
| 3000 | 402 | 503 | 604 | 704 | 805 | 906 | 1006 | 1107 | 1207 | 1308 | 1409 | 1509 |
| 3500 | 548 | 685 | 822 | 959 | 1096 | 1233 | 1370 | 1507 | 1643 | 1780 | 1917 | 2054 |
| 4000 | 716 | 894 | 1073 | 1252 | 1431 | 1610 | 1789 | 1968 | 2147 | 2325 | 2504 | 2683 |
| 4500 | 906 | 1132 | 1358 | 1585 | 1811 | 2038 | 2264 | 2490 | 2717 | 2943 | 3170 | 3396 |
| 5000 | 1118 | 1398 | 1677 | 1957 | 2236 | 2516 | 2795 | 3075 | 3354 | 3634 | 3913 | 4193 |
| 5500 | 1353 | 1691 | 2029 | 2367 | 2706 | 3044 | 3382 | 3720 | 4058 | 4397 | 4735 | 5073 |
| 6000 | 1610 | 2012 | 2415 | 2817 | 3220 | 3622 | 4025 | 4427 | 4830 | 5232 | 5635 | 6037 |
| 8500 | 1889 | 2362 | 2834 | 3306 | 3779 | 4251 | 4724 | 5196 | 5668 | 6141 | 6613 | 7085 |
| 7000 | 2191 | 2739 | 3287 | 3835 | 4383 | 4930 | 5478 | 6026 | 6574 | 7122 | 7669 | 8217 |
| 7500 | 2516 | 3144 | 3773 | 4402 | 5031 | 5660 | 6289 | 6918 | 7547 | 8175 | 8804 | 9433 |
| 8000 | 2862 | 3578 | 4293 | 5009 | 5724 | 6440 | 7155 | 7871 | 8586 | 9302 | 10017 | 10733 |
| 8500 | 3231 | 4039 | 4847 | 5654 | 6462 | 7270 | 8078 | 8885 | 9693 | 10501 | 11309 | 12116 |
| 9000 | 3622 | 4528 | 5433 | 6339 | 7245 | 8150 | 9056 | 9961 | 10867 | 11773 | 12678 | 13584 |
| 9500 | 4036 | 5045 | 6054 | 7063 | 8072 | 9081 | 10090 | 11099 | 12108 | 13117 | 14126 | 15135 |
| 10000 | 4472 | 5590 | 6708 | 7826 | 8944 | 10062 | 11180 | 12298 | 13416 | 14534 | 15652 | 16770 |
| 10500 | 4930 | 6163 | 7396 | 8628 | 9861 | 11093 | 12326 | 13559 | 14791 | 16024 | 17256 | 18489 |
| 11000 | 5411 | 6764 | 8117 | 9469 | 10822 | 12175 | 13528 | 14881 | 16233 | 17586 | 18939 | 20292 |
| 11500 | 5914 | 7393 | 8871 | 10350 | 11828 | 13307 | 14786 | 16264 | 17743 | 19221 | 20700 | 22178 |
| 12000 | 6440 | 8050 | 9660 | 11269 | 12879 | 14489 | 16099 | 17709 | 19319 | 20929 | 22539 | 24149 |
| 13000 | 7558 | 9447 | 11337 | 13226 | 15115 | 17005 | 18894 | 20784 | 22673 | 24562 | 26452 | 28341 |
| 13500 | 8150 | 10188 | 12225 | 14263 | 16300 | 18338 | 20376 | 22413 | 24451 | 26488 | 28526 | 30563 |
| 14000 | 8765 | 10956 | 13148 | 15339 | 17530 | 19722 | 21913 | 24104 | 26295 | 28487 | 30678 | 32869 |

EP 3 296 017 A1

$$\sqrt{\frac{\text{G- force}}{(1.118 \times 10^{-6})\ (\text{Radius in mm})}} = \text{RPM}$$

For accurate conversion skilled in the art knows that the abovementioned formula is used.

After blood aspiration and before centrifugation, the transparent calibrated tube/4/ will be seen as presented on the figure 4

The Calibrated transparent tube /4/, according to the invention is circular in cut section through the three formed chambers. The inner diameter of the lower / 4c / and upper / 4a / chamber of the calibrated transparent tube / 4 / is preferably uniform and equal to 14 mm, which provides a volume of 1,54 ml per 1 cm length of the tube.

Specifically we state that:

$$\text{Volume (ml)} = \pi r^2 (\text{cm}) \times \text{height (cm)},$$

Accordingly:

$$\text{Volume} = {}^{22}/_{7} \times (0.7)^2 \times 1 = 1.54 \text{ ml / cm length}$$

$$\text{Upper chamber volume} = 1.54 \times 3.5 \text{ cm} = 5.39 \text{ ml}$$

$$\text{Lower chamber volume} = 1.54 \times 2 \text{ cm} = 3.08 \text{ ml}$$

The wall /5/ of the middle chamber /4b/ of the calibrated transparent tube /4/, is thickened wall, interiorly coated by platelets antiadhesive coat to allow maximum platelets aspiration from the tube by minimizing platelets adhesions to the interior wall of the tube. The internal diameter of the upper /4a/ И lower chambers /4c/ is preferably about 7mm, which allows a volume of 0,385 ml for each 1cm tube length. Thus, in the middle chamber /4b/ (with length 4 cm) there will becollected 1,54 ml. For accuracy purposes, we state, that:

$$\text{volume (ml)} = \pi r^2 \text{ (cm)} \times \text{height (cm)}$$

$$= {}^{22}/_{7} \times (0.35)^2 \times 1 = 0.385 \text{ ml /cm length}$$

$$\text{Lower chamber volume} = 0.385 \times 4 \text{ cm} = 1.54 \text{ ml}$$

$$\text{Total volume} = 5.39 \text{ (upper)} + 1.54 \text{ (middle)} + 3.08 \text{ (lower)} = 10.01 \text{ ml (approximately 10 ml including 1 ml ACD anti-coagulant)}$$

Preferably, the middle chamber / 4b / of the calibrated transparent tube / 4 / was provided with four openings / 6 /, which are closed with four rubber stoppers / 7 /, wherein the openings / 6 / are designed to fit into the thick wall of the middle chamber / 4b / of the transparent calibrated tube / 4 /, at positions 3, 6, 9 and 12 hours on the clock dial, at 4 different levels in a helical manner to ensure the strength of the wall The thickness of the middle chamber / 4b / of the transparent calibrated tube / 4 / is within the range 2-3 mm, and the diameter of each opening is preferably about 2 mm (to allow the entry of a 16-gauge needle).

The rubber stoppers / 7 /, which are arranged helically on a plurality of levels allowing 16 gauge needle to be used for the collection of PRP buffer layer directly and effectively from the the side wall / 5 / of the middle chamber / 4b / of the calibrated transparent tube / 4 / (independently at what level was formed).

It will be clear to those skilled in the art that there may be many scenarios of how calibrated transparent tube / 4 / and PRP buffer layer will look after centrifugation, depending on the number and size of red blood cells (RBC) as well as the platelet count

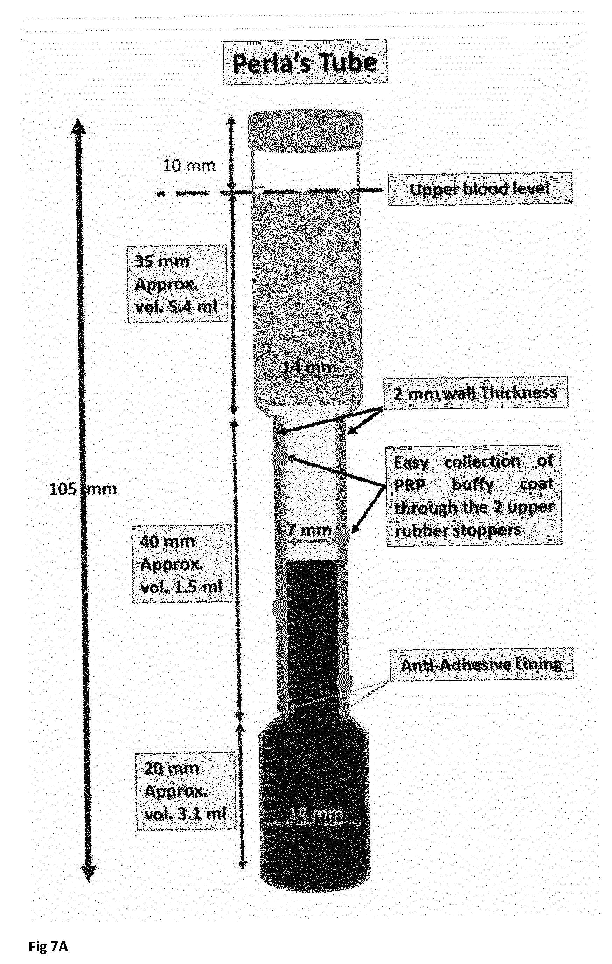

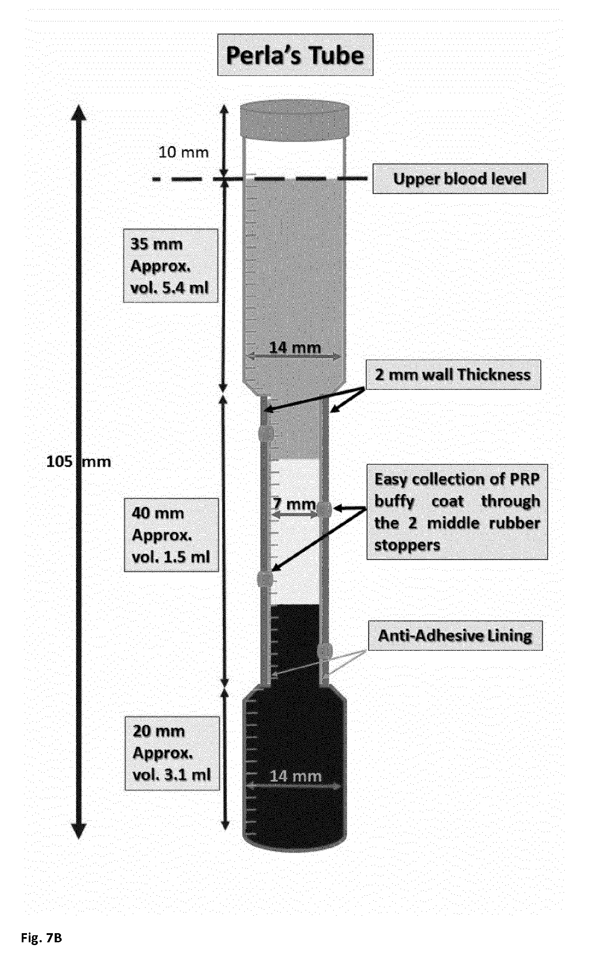

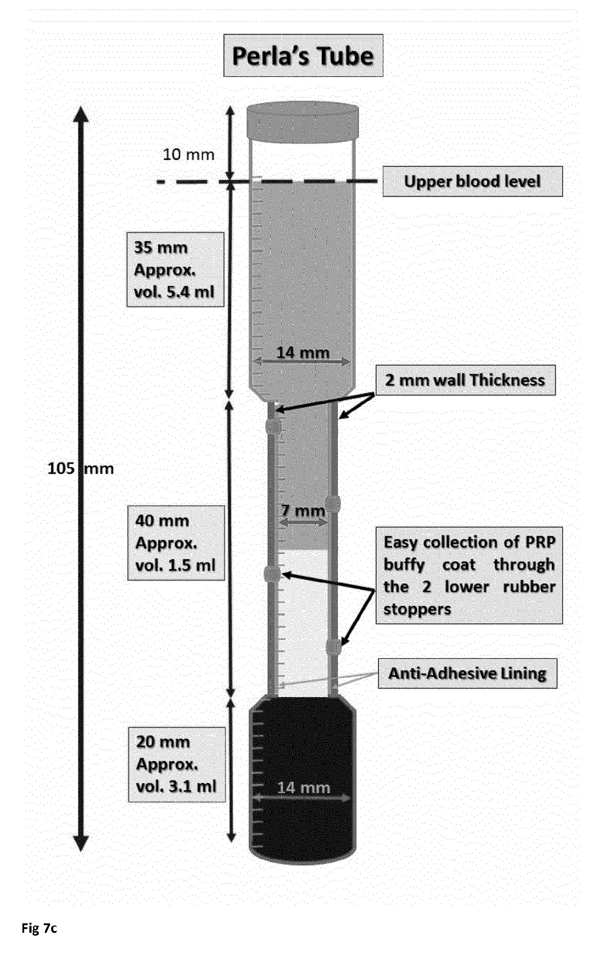

For a complete understanding of the effect of the modification made by the inventors of the present invention, three examples of scenarios are illustrated in Figures 7a-7c. For better understanding we specify that Figure 7a presents scenario in the case of patients with a high number and / or high volume of erythrocytes (red blood cells). FIG. 7b presents the scenario in the case of patients with an average number and / or average volume of erythrocytes (red blood cells). Accordingly, FIG. 7c presents the scenario in the case of patients with low and / or low volume of red blood cells (RBCs).

Alternatively, the tube / 1 / and the transparent calibrated tube / 4 / are provided in a kit accompanied with instructions as to obtain platelet-rich plasma. The three elements of the kit are placed inside a box, divided into two sections, each corresponding to the size and the shape of the tube/1/ and the transparent calibrated tube / 4 /, respectively.

The following table present results achieved in 8 cases where the collection of PRP was done from the stoppers /7/ in the middle chamber / 4b / of the calibrated transparent tube / 4 / after centrifugation when using wide 16 gauge needle:

| Case no. | Avg | 1 | 2 | 3 | 4 | 5 | 6 | 7 | 8 |
|---|---|---|---|---|---|---|---|---|---|
| Age | | 32 | 43 | 37 | 39 | 46 | 54 | 51 | 62 |
| Gender | | M | F | F | M | F | M | F | M |
| Blood PLT count (X10$^3$/ml) | 221.13 | 219.00 | 223.00 | 251.00 | 162.00 | 265.00 | 217.00 | 155.00 | 277.00 |
| Blood volume collected (ml) | 8.11 | 8.50 | 8.20 | 7.80 | 8.70 | 8.30 | 8.10 | 7.40 | 7.90 |
| PRP volume (ml) | 2.64 | 2.80 | 2.60 | 2.50 | 3.00 | 2.70 | 2.60 | 2.30 | 2.60 |
| PPP volume (ml) | 3.38 | 3.60 | 3.50 | 3.20 | 3.60 | 3.50 | 3.40 | 3.00 | 3.20 |
| Whole Blood-Total PLT | 1793.73 | 1861.50 | 1828.60 | 1957.80 | 1409.40 | 2199.50 | 1757.70 | 1147.00 | 2188.30 |
| PLT count in PPP (X103/ml) | 58.50 | 68.00 | 83.00 | 65.00 | 44.00 | 58.00 | 53.00 | 43.00 | 54.00 |
| Total PLT count in PPP (ml) | 198.34 | 244.80 | 290.50 | 208.00 | 158.40 | 203.00 | 180.20 | 129.00 | 172.80 |
| PRP: PLT count (X103/ml) | 502.25 | 489.00 | 496.00 | 532.00 | 415.00 | 596.00 | 533.00 | 375.00 | 582.00 |
| PRP: PLT-total count | 1325.56 | 1369.20 | 1289.60 | 1330.00 | 1245.00 | 1609.20 | 1385.80 | 862.50 | 1513.20 |
| Total PLT (PPP+PRP) | 1523.90 | 1614.00 | 1580.10 | 1538.00 | 1403.40 | 1812.20 | 1566.00 | 991.50 | 1686.00 |
| PPP + PRP% Recovery | 85.78 | 86.70 | 86.41 | 78.56 | 99.57 | 82.39 | 89.09 | 86.44 | 77.05 |
| PRP% Recovery | 74.59 | 73.55 | 70.52 | 67.93 | 88.34 | 73.16 | 78.84 | 75.20 | 69.15 |
| PRP PLT Concentration (folds) | 2.30 | 2.23 | 2.22 | 2.12 | 2.56 | 2.25 | 2.46 | 2.42 | 2.10 |

EP 3 296 017 A1

It was found by the current inventors, that the collection and recovery of the PRP carried out with the tube and the calibrated transparent tube, according the invention resulted in an improved more reliable end product due to:

1. Narrow lumen of the middle chamber; which allows better detection and vision ot PRP buffy coat volume through a lengthy in the range of 40 mm middle chamber.

2. Side wall /5/ stoppers /7/ on the middle chamber /4b/ allowing direct collection with wide needle, preferably 16-gauge needle, which minimize loss of PRP during aspiration from upper end by narrow gauge long needle.

3. Platelets anti-adhesive lining of the inner wall of the middle chamber/4b/, minimizing the adhesion of the platelets to the inner wall of the tube, during collection.

4. The Calibrated transparent tube/4/ was designed, so that it fits in all standard centrifuge devices.

In addition it should be noted that the tube and the calibrated transparent tube for fractionation may be used for collection and processing of blood for the preparation of autologous PRP (plasma rich in platelets) / PPP (platelet poor plasma) for medical and cosmetic indications, e.g. in medical face lifting ; in transplantation of hair during cutting and preparation of the grafts; after laser resurfacing; in care after burns; in fast healing indications; in post-operative wound healing; in proliferative therapy of articular inflammatory conditions; in care for chronic ulcers etc.

**Claims**

**1.**    A collection tube made of plastic material with a wide inner diameter and short length, preferably up to 20 cm, internally coated with a platelet anti-adhesive, **characterized in that** it is fitted with a needle / 2 / at one end of the tube / 1 / which is preferably 23- gauge sharp needle cannula with a butterfly-fixator for blood collection by venipuncture and needle / 3 / in the opposite end of the tube / 1 / which is 16- gauge sharp needle.

**2.**    A calibrated transparent tube with circular cross section made up of a capped upper chamber, middle chamber and a lower chamber, **characterized in that** middle chamber / 4b / is provided with openings / 5 /, distributed alternately on the wall / 6 / in consecutive levels, and every opening is equipped with stopper / 7 / made of flexible material for drilling of 16-gauge sharp needle, wherein the diameter of the upper chamber / 4a/ and the lower chamber / 4b / are equal and are two times greater than the diameter of the middle chamber / 4b /, wherein the working volume of the upper chamber / 4a / is 5.39 ml, of middle chamber / 4b / is 1.54 ml and of lower chamber is 3.08 ml, and the diameter of the middle chamber is provided by inward slopes of the lower end of the upper chamber / 4a / and the upper end of the lower chamber / 4c /.

**3.**    A calibrated transparent tube, according to claim 2, wherein, the openings /5/ are alternately distributed on the wall /6/ in an imaginary helix successively in positions 3, 6, 9 and 12 hours of clock dial.

**4.**    A kit for preparation of platelet rich plasma (PRP) for use in treating a subject in need thereof, wherein the kit comprises:

- collection tube, according to claim 1;
- transparent calibrated tube, according to claim 2; and
- written instructions for the preparation of platelet rich plasma,
wherein the elements, are placed in a box, divided into two sectors, each with size and shape corresponding to the tube /1/ and tube/4/

**Amended claims in accordance with Rule 137(2) EPC.**

**1.**    A calibrated transparent tube with circular cross section made up of a capped upper chamber, middle chamber and a lower chamber, **characterized in that** middle chamber / 4b / is provided with openings / 5 / distributed alternately on the wall / 6 / in zig-zag cross sectional order, and every opening is equipped with stopper / 7 / made of flexible material for drilling of 16-gauge sharp needle, wherein the diameter of the upper chamber / 4a/ and the lower chamber / 4b / are equal and are two times greater than the diameter of the middle chamber / 4b /, wherein the working volume of the upper chamber / 4a / is 5.39 ml, of middle chamber / 4b / is 1.54 ml and of lower chamber is 3.08 ml, and the diameter of the middle chamber is provided by inward slopes of the lower end of the upper chamber / 4a / and the upper end of the lower chamber / 4c /.

2. A calibrated transparent tube, according to claim 1, wherein, the openings /5/ are alternately distributed on the wall /6/ in an imaginary helix successively in positions 3, 6, 9 and 12 hours of clock dial.

3. A kit for preparation of platelet rich plasma (PRP) for use in treating a subject in need thereof, wherein the kit comprises:

- collection tube, made of plastic material with a wide inner diameter and short length, preferably up to 20 cm, internally coated with a platelet anti-adhesive, **characterized in that** it is fitted with a needle / 2 / at one end of the tube / 1 / which is preferably 23-gauge sharp needle cannula with a butterfly-fixator for blood collection by venipuncture and needle / 3 / in the opposite end of the tube / 1 / which is 16- gauge sharp needle;
- transparent calibrated tube, according to claim 2; and
- written instructions for the preparation of platelet rich plasma,

wherein the elements, are placed in a box, divided into two sectors, each with size and shape corresponding to the tube /1/ and tube/4/

**Fig. 1**

Fig. 2

Fixed Angle

Radius of device

Swinging Bucket

Swinging bucket
running position

Radius of device

**Fig. 3**

The transparent calibrated tube before centrifugation

**Perla's Tube**

10 mm

Upper blood level

35 mm
Approx.
vol. 5.4 ml

14 mm

2 mm wall Thickness

105 mm

40 mm
Approx.
vol. 1.5 ml

4 rubber stoppers; each is 1.5 - 2 mm diameter distributed in 3,6,9,12 clock pattern

Anti-Adhesive Lining

20 mm
Approx.
vol. 3.1 ml

14 mm

Fig. 4

Cut Section of middle part
of Tube with narrow lumen

Fig. 5A

Cut Section of middle part
of Tube with narrow lumen

Cut Section of middle part
of Tube with narrow lumen

Cut Section of middle part
of Tube with narrow lumen

Cut Section of middle part
of Tube with narrow lumen

Fig 5B

Volume scheme of the transparent
calibrated tube before centrifugation

10 mm

10 ml

9 ml

8 ml

7 ml

6 ml

5 ml

7 mm

4 ml

7 mm

3ml

2 ml

1 ml

35 mm
Approx.
vol. 5.4
ml

105 mm

40 mm
Approx.
vol. 1.5
ml

20 mm
Approx.
vol. 3.1
ml

large diam. Section
10 mm length =
1.54 ml volume

14 mm

Small diam. Section
10 mm length =
0.38 ml volume

7 mm

7 mm

14 mm

**Fig 6**

**Perla's Tube**

10 mm

Upper blood level

35 mm
Approx.
vol. 5.4 ml

14 mm

2 mm wall Thickness

105 mm

Easy collection of PRP buffy coat through the 2 upper rubber stoppers

7 mm

40 mm
Approx.
vol. 1.5 ml

Anti-Adhesive Lining

20 mm
Approx.
vol. 3.1 ml

14 mm

Fig 7A

Fig. 7B

Fig 7c

Europäisches
Patentamt

European
Patent Office

Office européen
des brevets

# EUROPEAN SEARCH REPORT

Application Number

EP 16 18 9473

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2009/209933 A1 (ZYLBERBERG JAVIER [US] ET AL) 20 August 2009 (2009-08-20) * paragraph [0001] * | 1 | INV. B01L3/00 A61B5/15 |
| Y | CN 201 524 083 U (TIANJIN BAILI KANGTAI BIOTECHNOLOGY CO LTD) 14 July 2010 (2010-07-14) | 1 | |
| A | * paragraph [0019]; figure 1 * | 4 | |
| A | US 2010/168535 A1 (ROBINSON MARK RIES [US] ET AL) 1 July 2010 (2010-07-01) * paragraph [0053] * | 1 | |
| A | US 6 077 235 A (SERPENTINO PETER [US] ET AL) 20 June 2000 (2000-06-20) * column 1, lines 39-50 * | 1 | |
| A | WO 2014/165498 A1 (ALLIANCE PARTNERS LLC [US]) 9 October 2014 (2014-10-09) * paragraphs [0019] - [0026]; figure 1 * | 2-4 | |
| A | KR 101 409 443 B1 (KIM JUN WOO [KR]) 20 June 2014 (2014-06-20) * figure 7 * | 2-4 | TECHNICAL FIELDS SEARCHED (IPC) B01L A61B |

The present search report has been drawn up for all claims

| Place of search | Date of completion of the search | Examiner |
|---|---|---|
| The Hague | 28 February 2017 | Campbell, Paul |

EPO FORM 1503 03.82 (P04C01)

**ANNEX TO THE EUROPEAN SEARCH REPORT
ON EUROPEAN PATENT APPLICATION NO.**                    EP 16 18 9473

This annex lists the patent family members relating to the patent documents cited in the above-mentioned European search report.
The members are as contained in the European Patent Office EDP file on
The European Patent Office is in no way liable for these particulars which are merely given for the purpose of information.

28-02-2017

| Patent document cited in search report | | | Publication date | Patent family member(s) | | | Publication date |
|---|---|---|---|---|---|---|---|
| US 2009209933 | A1 | | 20-08-2009 | NONE | | | |
| CN 201524083 | U | | 14-07-2010 | NONE | | | |
| US 2010168535 | A1 | | 01-07-2010 | NONE | | | |
| US 6077235 | A | | 20-06-2000 | DE | 60012599 | D1 | 09-09-2004 |
| | | | | DE | 60012599 | T2 | 04-08-2005 |
| | | | | EP | 1031380 | A2 | 30-08-2000 |
| | | | | JP | 3364609 | B2 | 08-01-2003 |
| | | | | JP | 2000271106 | A | 03-10-2000 |
| | | | | US | 6077235 | A | 20-06-2000 |
| | | | | US | 6290655 | B1 | 18-09-2001 |
| WO 2014165498 | A1 | | 09-10-2014 | EP | 2981357 | A1 | 10-02-2016 |
| | | | | US | 2015004080 | A1 | 01-01-2015 |
| | | | | US | 2016129438 | A1 | 12-05-2016 |
| | | | | WO | 2014165498 | A1 | 09-10-2014 |
| KR 101409443 | B1 | | 20-06-2014 | NONE | | | |

EPO FORM P0459

For more details about this annex : see Official Journal of the European Patent Office, No. 12/82

**EP 3 296 017 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 201020251411 A